Europäisches Patentamt

(19) European Patent Office

Office européen des brevets

(11) Publication number: **0 092 867**
**B1**

(12) **EUROPEAN PATENT SPECIFICATION**

(45) Date of publication of patent specification: **16.07.86**

(21) Application number: **83200544.1**

(22) Date of filing: **15.04.83**

(51) Int. Cl.⁴: **C 07 C 49/403,** C 07 C 35/08, C 07 C 45/33, C 07 C 45/53, C 07 C 29/50, C 07 C 29/132

(54) Process for preparing cyclohexanol and cyclohexanone.

(30) Priority: **23.04.82 NL 8201695**

(43) Date of publication of application:
**02.11.83 Bulletin 83/44**

(45) Publication of the grant of the patent:
**16.07.86 Bulletin 86/29**

(84) Designated Contracting States:
**BE CH DE FR GB IT LI NL**

(56) References cited:
**FR-A-2 140 088**
**FR-A-2 313 336**
**US-A-4 238 415**

The file contains technical information
submitted after the application was filed and
not included in this specification

(73) Proprietor: **STAMICARBON B.V.**
**Mijnweg 1**
**NL-6167 AC Geleen (NL)**

(72) Inventor: **Housmans, Johannes Gerardus H. M.**
**St. Joosterweg 22**
**NL-6051 HE Maasbracht (NL)**
Inventor: **Van Nispen, Simon Petrus Joahnnes M.**
**Erenfriedstrasse 8**
**NL-6191 SJ Beek (NL)**
Inventor: **Plantema, Otto Gerrit**
**Braakpeel 1**
**NL-6034 RP Nederweert Eind (NL)**

(74) Representative: **Hatzmann, Marinus Jan et al**
**OCTROOIBUREAU DSM P.O.Box 9**
**NL-6160 MA Geleen (NL)**

Courier Press, Leamington Spa, England.

## Description

The invention relates to a process for preparing cyclohexanol and cyclohexanone by oxidizing cyclohexane with a gas containing molecular oxygen to form an oxidation mixture containing cyclohexylhydroperoxide and treating the oxidation mixture with a metal salt in the presence of an aqueous solution of an alkalimetalhydroxide for the decomposition of the cyclohexylhydroperoxide.

Such a process can be performed in the manner described in the United Kingdom patent specification 1,382,849, in which the decomposition of the hydroperoxide is effected at a temperature of 80—170°C, for instance 135 or 145°C and a pH of the aqueous phase in the reaction mixture of between 8 and 13, preferably between 9 and 11, measured at 25°C.

According to the United States patent specification 4,238,415 relating to the decrease in the consumption of lye in this known process, the decomposition of the hydroperoxide is effected also at a temperature of 80—170°C, but in the presence of such a quantity of alkalimetalhydroxide that the OH⁻-concentration in the aqueous phase after the decomposition is preferably 0.6—1 N. It has now been found that the decomposition of the hydroperoxide can be effected in such a manner as to give a higher yield of cyclohexanol and cyclohexanone.

The process according to the invention for preparing cyclohexanol and cyclohexanone by oxidizing cyclohexane with a gas containing molecular oxygen to form an oxidation mixture containing cyclohexylhydroperoxide and treating the oxidation mixture with a metal salt in the presence of an aqueous solution of an alkalimetalhydroxide for the decomposition of the cyclohexylhydroxide is characterized in that the treatment of the oxidation mixture is effected at a temperature of 70—115°C.

Said treatment can be carried out e.g. in a stirred tank reactor. By preference said treatment is carried out in a countercurrent column, e.g. a rotating disc contactor, because in such a column a more rapid decomposition of the peroxide with less alkalimetalhydroxide can be achieved. The chosen quantity of alkalimetalhydroxide to be used may differ, for instance, when using a stirred tank reactor, such a quantity that the OH⁻-concentration in the resulting aqueous phase after the decomposition is 0.5—2 N, preferably 1—1.8 N. When a countercurrent column is used, said OH⁻ concentration is preferably below 0.1 N, most advantageously below 0.01 N.

In applying the process according to the invention the oxidation of the cyclohexane is effected as known in the art in the liquid phase with, for instance, air, pure oxygen or a mixture of oxygen and inert gas at temperatures of 120—200°C, specifically 140—180°C. In this process, for instance 1—12% of the cyclohexane is converted. In this oxidation process the pressure is not critical and is mostly between 1 and 50 bar, for instance 13 bar.

The oxidation of the cyclohexane is preferably effected in the absence of substances promoting the decomposition of the cyclohexylhydroperoxide formed, such as compounds of transition metals, and that is why for this oxidation process a reactor is used having an inert inner wall, for instance an inner wall of passivated steel, aluminium, glass, enamel and similar materials. If it is desired yet to use an oxidation catalyst, the quantity should preferably be very small, for instance in the order of 1 part by weight per million. As oxidation catalyst compounds of, for instance, cobalt, chromium, manganese, iron, nickel or copper can be used. Particularly suitable are cobaltous naphthenate and cobalt-2-ethyl-hexanoate.

The decomposition of the cyclohexylhydroperoxide in the oxidation mixture can be effected by means of metal salts, for instance salts of transition metals such as cobalt, nickel, iron, chromium, manganese and copper. Preference is given to effecting this decomposition with, as metal salt, a salt of cobalt and/or of chromium, for instance a sulphate or nitrate. The chosen quantity of metal salt may differ, for instance a quantity of 0.1—1000 parts by weight per million (calculated as metal and with respect to the aqueous phase). Very suitable is a quantity of metal salt of 1—200 parts by weight per million. The metal salt can be added in the form of an aqueous solution, optionally together with the alkalimetalhydroxide, to the oxidation mixture. The metal can be added also to the oxidation mixture as a salt of an organic acid dissolved in an organic solvent, for instance cyclohexane. The ratio between the aqueous phase and the organic phase in the decomposition of the cyclohexylhydroperoxide is preferably chosen between 0.005 and 1.5 litres aqueous phase per litre organic phase obtained, specifically between 0.01 and 0.5 litre. During the decomposition the desired temperature within the range of 70—115°C is preferably maintained by applying reflux cooling and/or cooling of the input organic phase. No recirculation of evaporated products will then be needed, which has a slightly favourable effect on the yield of desired product. In the decomposition the chosen pressure is usually slightly lower than in the oxidation.

Before the decomposition of the hydroperoxide in the oxidation mixture, the oxidation mixture can be treated, if so desired, with an aqueous alkalimetalhydroxide or alkalimetalcarbonate solution for the purpose for neutralizing the acids formed in the oxidation process, for instance to a pH of the aqueous phase of 8—13. When applying such neutralization, the total lye consumption can be reduced in the manner described in the United States patent specification 4,238,415 by applying in the neutralization the aqueous phase that can be separated off from the reaction mixture after the decomposition. When the decomposition is carried out in a countercurrent column, said neutralization can be performed in the lower part of the column.

The reaction mixture obtained in the decomposition of the hydroperoxide can be processed further by separating off the aqueous phase and subjecting the organic phase, if so desired after washing with water, to a distillation while recovering cyclohexane to be returned, as well as cyclohexanol and cyclohexanone. The cyclohexanol and cyclohexanone thus obtained are found to be pure enough, without further treatment, for further conversion into caprolactam (monomer of nylon 6), which constitutes a major advantage of the process according to the invention compared with a decomposition of cyclohexylhydroperoxide at 135 or 145°C at a pH of between 8 and 13 according to the process described in the United Kingdom patent specification 1,382,849.

In the following examples the process according to the invention will be further elucidated.

## Example I

In an oxidation reactor having a capacity of 13 litres cyclohexane is oxidized by means of air at 170°C and 13 bar while being stirred. Per hour 43 kg cyclohexane is fed to the reactor and 2000 litres air is passed through. Of the cyclohexane 1.2 kg is converted per hour.

The oxidation mixture thus obtained (per kg it contains 29 g cyclohexylhydroperoxide, cyclohexanol and cyclohexanone, as was as 45 milligramme equivalents of acids) is pumped into a decomposition reactor having a capacity of 13 litres and treated at a pressure of 1.5 bar and 110°C, during reflux cooling, with an aqueous cobaltous sulphate solution (20 parts by weight per million) and sodium hydroxide solution (14 % wt). Per hour 4.5 litres cobaltous sulphate solution and 4.5 litres sodium hydroxide solution are fed to the decomposition reactor. The reaction mixture obtained from the decomposition reactor is separated into an organic phase and an aqueous phase (OH$^-$-concentration 1.5 gramme equivalents per litre). The organic phase is separated by distillation into cyclohexane, which is returned to the oxidation reactor, and cyclohexanol and cyclohexanone. With a complete conversion of the hydroperoxide in the decomposition reactor, 1242 g cyclohexanol and cyclohexanone is obtained per hour.

## Example II

Cyclohexanol and cyclohexanone are prepared in the same way as in example I, but with such a quantity of lye in the decomposition reactor that the OH$^-$-concentration in the aqueous phase obtained is 0.7 gramme equivalents per litre. Per hour 1229 g cyclohexanol and cyclohexanone is obtained.

## Comparative examples

Cyclohexanol and cyclohexanone are prepared in the same way as in Example I, but at a temperature in the decomposition reactor of 135, respectively 155°C. Per hour only 1190, respectively 1179 g, cyclohexanol and cyclohexanone is then obtained.

## Example III

Cyclohexanol and cyclohexanone are prepared in the same way as in Example 1 while applying a temperature of 85°C in the decomposition reactor under otherwise the same conditions 1260 g cyclohexanol and cyclohexanone is obtained. Similar yields are obtainable at decomposition temperatures below 85°C but long residence times and/or large reaction volumes are necessary to obtain complete decomposition at temperatures below 70°C.

## Example IV

Cyclohexanol and cyclohexanone are prepared in the way described in Example I. But instead of an aqueous cobaltous sulphate solution an aqueous chromic nitrate solution (150 parts by weight per million) is applied, while in the decomposition reactor the temperature is maintained at 105°C (pressure about 1.3 bar). 1257 g cyclohexanol and cyclohexanone is obtained.

## Example V

Cyclohexanol and cyclohexanone are prepared in the same way as described in Example I, however with an aqueous solution containing cobaltous sulphate (10 parts by weight per million) and chromic nitrate (20 parts by weight per million) while in the decomposition reactor the temperature is maintained at 85°C. Per hour, 1274 g cyclohexanol and cyclohexanone is obtained.

## Example VI

To the bottom of a rotating disc contactor (as described in American Institute of Chem. Eng. Journal vol. 8 pages 252—261, 1962) having an effective height of 3.5 m and a diameter of 8 cm, is supplied 150 kg per hour of an oxidation mixture of the same composition as in Example I.

To the top of said contactor, 1.5 l per hour of an aqueous solution of sodium hydroxide (14% wt) and 1.5 l per hour of an aqueous solution of cobaltous sulphate (20 parts by weight per million) is supplied. By adjusting the stirrer speed of the rotating shaft, the hold up of the aqueous phase in the contactor is maintained at 3—5 %. The temperature in the contactor is 85°C and the pressure 1.5 bar. The organic phase leaving the contactor is free of peroxide. The pH of the aqueous phase leaving the contactor is kept at 8—10 (OH$^-$-concentration $10^{-6}$—$10^{-4}$ N). The organic phase obtained is worked up in the same way as in Example I. Per hour 4390 g of cyclohexanol and cyclohexanone is obtained.

## Claims

1. Process for preparing cyclohexanol and cyclohexanone by oxidizing cyclohexane with a gas containing molecular oxygen to form an oxidation mixture containing cyclohexylhydroperoxide and treating the oxidation mixture with a metal salt in the presence of an aqueous solution in an alkalimetalhydroxide for the decomposition of the cyclohexylhydroperoxide, characterized in that the treatment of the oxidation mixture is effected at a temperature of 70—115°C.

2. Process according to claim 1, characterized in that the treatment of said oxidation mixture is carried out in a countercurrent column.

3. Process according to claim 2, characterized in that the quantity of alkalimetalhydroxide employed in treating said oxidation mixture is such that the OH⁻-concentration in the resulting aqueous phase after said composition is below 0.01 N.

4. Process according to claim 1, characterized in that the quantity of alkalimetalhydroxide used in the treatment of the oxidation mixture in a stirred tank reactor is such that the OH⁻-concentration in the resulting aqueous phase after the decomposition is 1—1.8 N.

5. Process according to any one of claims 1—4, characterized in that the metal salt used in the treatment of the oxidation mixture is a salt of cobalt and/or of chromium.

6. Process according to any one of claims 1—5, characterized in that 1—200 parts by weight per million metal salt are used, calculated as metal and with respect to the aqueous phase.

7. Process according to any one of claims 1—6, characterized in that, in the treatment of the oxidation mixture the desired temperature is maintained by applying reflux cooling and/or cooling of the oxidation mixture to be treated.

8. Process according to any one of claims 1—7, characterized in that the treatment of the oxidation mixture is effected while applying 0.01—0.5 litre aqueous phase per litre organic phase obtained.

## Patentansprüche

1. Verfahren zur Herstellung von Cyclohexanol und Cylohexanon durch Oxidieren von Cyclohexan mit einem molekularen Sauerstoff enthaltenden Gas unter Bildung eines Cyclohexylhydroperoxid enthaltenden Oxidationsgemisches und Behandlung des Oxidationsgemisches mit einem Metallsalz in Gegenwart einer wässerigen Lösung eines Alkalimetallhydroxids für die Zersetzung des Cyclohexylhydroperoxids, dadurch gekennzeichnet, daß die Behandlung des Oxidationsgemisches bei einer Temperatur von 70—115°C durchgeführt wird.

2. Verfahren nach Anspruch 1, dadurch gekennzeichnet, daß die Behandlung des Oxidationsgemisches in einer Gegenstromkolonne durchgeführt wird.

3. Verfahren nach Anspruch 2, dadurch gekennzeichnet, daß für die Behandlung des Oxidationsgemisches eine solche Menge Alkalimetallhydroxid verwendet wird, daß die OH⁻-Konzentration in der resultierenden wässerigen Phase nach der Zersetzung weniger als 0,01 N beträgt.

4. Verfahren nach Anspruch 1, dadurch gekennzeichnet, daß für die Behandlung des Oxidationsgemisches in einem gerührten Tankreaktor eine solche Menge Alkalimetallhydroxid verwendet wird, daß die OH⁻-Konzentration in der resultierenden wässerigen Phase nach der Zersetzung 1—1,8 N beträgt.

5. Verfahren nach einem der Ansprüche 1—4, dadurch gekennzeichnet, daß das für die Behandlung des Oxidationsgemisches verwendet Metallsalz ein Salz von Kobalt und/oder Chrom ist.

6. Verfahren nach einem der Ansprüche 1—5, dadurch gekennzeichnet, daß 1—200 Gewichtsteile je Million Metallsalz verwendet werden, berechnet als Metall und bezogen auf die wässerige Phase.

7. Verfahren nach einem der Ansprüche 1—6, dadurch gekennzeichnet, daß bei der Behandlung des Oxidationsgemisches die gewünschte Temperatur durch Anwendung von Rückflußkühlung und/oder Kühlung des zu behandelnden Oxidationsgemisches aufrechterhalten wird.

8. Verfahren nach einem der Ansprüche 1—7, dadurch gekenzeichnet, daß die Behandlung des Oxidationsgemisches unter Verwendung von 0,01—0,5 Liter wässeriger Phase je Liter erhaltener organischer Phase durchgeführt wird.

## Revendications

1. Procédé de préparation de cyclohexanol et de cyclohexanone par oxydation du cyclohexane avec un gaz contenant de l'oxygène moléculaire pour former un mélange d'oxydation contenant de l'hydroperoxyde de cyclohexyle et par traitement du mélange d'oxydation avec un sel métallique en présence d'une solution aqueuse d'un hydroxyde de métal alcalin pour décomposer l'hydroperoxyde de cyclohexyl, caractérisé en ce qu'on effectue le traitement du mélange d'oxydation à une température de 70 à 115°C.

2. Procédé selon la revendication 1, caractérisé en ce qu'on effectue le traitement dudit mélange d'oxydation dans une colonne à contrecourant.

3. Procédé selon la revendication 2, caractérisé en ce que la quantité d'hydroxyde de métal alcalin qu'on emploie pour traiter ledit mélange d'oxydation est telle que la concentration en OH⁻ dans la phase aqueuse résultante après ladite décomposition soit inférieure à 0,01 N.

4. Procédé selon la revendication 1, caractérisé en ce que la quantité de l'hydroxyde de métal alcalin utilisée dans le traitement du mélange d'oxydation alcalin utilisée dans le traitement du mélange d'oxydation dans un réacteur à cuve agitée est tele que la concentration en OH⁻ dans la phase aqueuse résultante après la décomposition soit de 1 à 1,8 N.

5. Procédé selon l'une quelconque des revendications 1 à 4, caractérisé en ce que le sel métallique utilisé dans le traitement du mélange d'oxydation est un sel de cobalt et/ou de chrome.

6. Procédé selon l'une quelconque des revendications 1 à 5, caractérisé en ce qu'on utilise 1 à 200 ppm en poids de sel métallique, calculé en métal et par rapport à la phase aqueuse.

7. Procédé selon l'une quelconque des revendications 1 à 6, caractérisé en ce que dans le traitement du mélange d'oxydation, on maintient la température désirée par application de refroidissement au reflux et/ou refroidissement du mélange d'oxydation à traiter.

8. Procédé selon l'une quelconque des revendications 1 à 7, caractérisé en ce qu'on effectue le traitement du mélange d'oxydation pendant qu'on applique 0,01 à 0,5 litre de phase aqueuse par litre de la phase organique obtenue.